# EUROPEAN PATENT APPLICATION

(11) **EP 4 361 632 A1**
(43) Date of publication of application: **01.05.2024**
(21) Application number: 22204805.0
(22) Date of filing: 31.10.2022
(51) Int. Cl.: G01N 33/543

(54) **ELECTROCHEMICAL LATERAL FLOW TEST DEVICE**

(71) Applicant: AIT Austrian Institute of Technology GmbH, 1210 Wien (AT)
(72) Inventor: HAINBERGER, Rainer, 1100 Wien (AT); MAIER, Thomas, 1230 Wien (AT); MUTINATI, Giorgio, 1050 Wien (AT); KOTHE, Tim, 1130 Wien (AT)
(74) Representative: SONN Patentanwälte GmbH & Co KG

(57) **Abstract**

A method for producing an electrochemical lateral flow test device (14) for the detection of a biomarker (7), the method comprising the steps:
- providing a porous substrate (4) enabling a capillary flow of a sample (6) comprising the biomarker (7) through the porous substrate (4);
- applying at least a working electrode (16) and a counter electrode (21) on the porous substrate (4); and
- depositing capture molecules (18, 18A, 18B, 18C, 18D) onto the porous substrate (4) next to the applied working electrode (16); and an electrochemical lateral flow test device (14).

## Description

The disclosure concerns a method for producing an electrochemical lateral flow test device for the detection of a biomarker.

The project leading to this application has received funding from the European Union's Horizon 2020 research and innovation programme under grant agreement No 761167, project name IMPETUS (PSP: 1.G4.00215.0.0).

Lateral flow test devices provide an easy-to-use and typically cost-efficient possibility to test a sample for the presence of a biomarker such as an antigen. The basic principle of lateral flow devices relies typically on an antigen-antibody reaction. In general, a sample runs along a porous substrate, which is equipped with a number of molecules, such as for example antibodies, that react with the antigen leading to a measurable output such as a change in color or a change of electrical properties. Involved molecules are typically referred to as conjugates and capture molecules. The currently most common lateral flow devices rely on a chemical reaction that leads to a change in color of a test strip. In case the color of the strip changes after a sample has been applied, there is evidence that the antigen is present in the sample. A well-known example are Covid-19 rapid tests. In the case of electrochemical lateral flow test devices, the readout is not based on a color change but on a change in electrical properties. This change in electrical properties can be detected by means of electrodes. Compared to the readout via the color change, electrochemical lateral flow devices are capable of delivering a more objective result and also facilitate quantitative assessment of the resulting signal.

Different electrochemical lateral flow devices have been proposed. For example, a disposable paper based microfluidic immunosensor based on screen printed electrodes is discussed in Cao, Liangli, et al. "A disposable paper-based microfluidic immunosensor based on reduced graphene oxide-tetraethylene pentamine/Au nanocomposite decorated carbon screen-printed electrodes." Sensors and Actuators B: Chemical 252 (2017): 44-54. In this paper, it is shown that pre-functionalized screen-printed electrodes can be assembled on a filter paper. An electrochemical reaction of the sample at the functionalized electrodes can be detected, thus the presence of the sample can be determined.

A further paper-based electrochemical immunosensor is known from: Zhu, Xuena, et al. "A paper electrode integrated lateral flow immunosensor for quantitative analysis of oxidative stress induced DNA damage." Analyst 139.11 (2014): 2850-2857. Screen printed electrodes are applied to a previously prepared lateral-flow strip.

Another electrochemical lateral flow immunosensor is known from Sinawang, Prima Dewi, et al. "Electrochemical lateral flow immunosensor for detection and quantification of dengue NS1 protein." Biosensors and Bioelectronics 77 (2016): 400-408. Biofunctionalized screen-printed gold electrodes are integrated in a cellulosic lateral flow strip. The biofunctionalization of the electrodes takes place before the electrodes are applied on the cellulosic lateral flow strip.

Petruzzi, Loric, et al. "Quantitative detection of C-reactive protein in human saliva using an electrochemical lateral flow device." Biosensors and Bioelectronics: X 10 (2022): 100136 shows another electrochemical lateral flow device. Electrodes are attached to dedicated holders, which allow a precise positioning of the electrodes with respect to a nitrocellulose membrane. The membrane includes immobilized capture antibodies.

At least the capture antibodies are sensitive towards temperature and consequently also sensitive towards pressure. This can have a negative impact on the reproducibility of such lateral flow devices as the application of the electrodes on the substrate can damage the involved molecules present in the substrate. Hence, the amount and density of undamaged and potentially active molecules is influenced by the application of the electrodes in an unpredictable way, introducing a significant source of uncertainty for any quantitative assessment. A further drawback of state of the art electrochemical lateral flow devices is that capture antibodies typically have to be applied to a relatively large area of for example a membrane. Therefore, also a relatively large amount of capture antibodies is needed, whereas only a fraction of it contributes to an electrical signal that can be measured by an electrode.

It is an object of the invention to alleviate or eliminate at least some disadvantages of the prior art. In particular it is an object of the invention to provide a method for producing an electrochemical flow device, which enables more reproducible quantitative results.

This is achieved by a method for producing an electrochemical lateral flow test device for the detection of a biomarker, the method comprising the steps:
- providing a porous substrate enabling a capillary flow of a sample comprising the biomarker through the porous substrate;
- applying at least a working electrode and a counter electrode on the porous substrate; and
- depositing capture molecules onto the substrate next to the applied working electrode.

Correspondingly, the object mentioned above can be achieved by an electrochemical lateral flow test device for the detection of a biomarker, the electrochemical lateral flow test device comprising:
- a porous substrate enabling a capillary flow of a sample comprising the biomarker through the porous substrate,
- at least a working electrode and a counter electrode for electrochemical detection arranged on the substrate,

wherein the porous substrate comprises capture molecules,
wherein the electrochemical lateral flow test device further comprises at least one access opening for depositing the capture molecules onto the substrate next to the previously applied working electrode through the at least one access opening during production of the electrochemical lateral flow test.

The electrochemical lateral flow test device can be used to detect the presence of a biomarker, such as for example an antigen, in the sample. The porous substrate may for example be a cellulose membrane, which allows capillary flow of the sample. The sample might for example be a biological liquid, such as blood, saliva or urine, and might additionally also be diluted in a buffer. The substrate is preferably hydrophilic. On top of the porous substrate, at least a counter electrode and a working electrode are applied. The working electrode ultimately is used to detect a change of the chemical composition in its environment due to a chemical reaction triggered by the biomarker. In general, the capture molecules are deposited into the substrate or onto the substrate next to the working electrode and optionally at least partially diffuse into the substrate during production or during use of the electrochemical lateral flow test device. The electrochemical lateral flow device comprises at least one access opening for depositing the capture molecules. For example, the top side of the porous substrate may be essentially unobstructed, therefore the unobstructed top side of the porous substrate may be the access opening. Alternatively, only a fraction of the porous substrate next to the working electrode might be unobstructed. The capture molecules may be deposited onto or next to the working electrode. The capture molecules can travel or diffuse into the porous substrate next to and/or underneath the applied working electrode. The deposition of the capture molecules is performed after applying the working electrode on the porous substrate. The capture molecules are deposited next to or in the vicinity of the previously applied working electrode. When depositing the capture molecules the working electrode is already present on the substrate. Hence, any changes in temperature or pressure that may be caused by the application of the electrode on the porous substrate, have happened (and have preferably vanished) before the capture molecules are deposited onto or into the porous substrate. The working electrode can be configured to detect a change in electrical properties in its environment in or within the porous substrate due to a chemical reaction involving at least the capture molecules, conjugate molecules as well as the targeted biomarker. Optionally, also substrate molecules undergoing a reaction mediated by an enzyme, which can be comprised in the conjugate molecules, can be part of the chemical reaction.

As in all kinds of immunoassays, the specificity of the response to a certain biomarker is achieved through the high binding strength between the biomarker and a specific antibody which is used as a capture antibody. The capture antibody can form at least a part of the capture molecule or form the capture molecule.

In one of the examples for electrochemical lateral flow devices mentioned above (Petruzzi, Loric, et al. "Quantitative detection of C-reactive protein in human saliva using an electrochemical lateral flow device." Biosensors and Bioelectronics: X 10 (2022): 100136), the biomarker is C-reactive protein (CRP), and capture molecules with a corresponding CRP-specific capture antibody are immobilized on the porous substrate. When the sample is applied to the electrochemical lateral flow device, the biomarker first binds to the conjugate molecules, and later on to the capture antibodies forming the capture molecules. The conjugate molecules comprise another CRP-specific capture antibody and an enzyme (alkaline phosphatase), which converts a substance in the environment of the working electrode (L-Ascorbic acid 2-phosphate sesquimagnesium salt hydrate) into another, electrochemically active substance (L-ascorbic acid), allowing the electrochemical quantification of the biomarker.

Another example for electrochemical lateral flow devices mentioned above (Zhu, Xuena, et al. "A paper electrode integrated lateral flow immunosensor for quantitative analysis of oxidative stress induced DNA damage." Analyst 139.11 (2014): 2850-2857.) illustrates the possibility to obtain a simplified assay. Again, the specificity of the assay is obtained by immobilizing a specific capture antibody for the biomarker (8-hydroxy-2'-deoxyguanosine). In this case, however, the biomarker itself is electrochemically active, which allows the direct quantification by measuring an electrochemical signal on the working electrode.

In order to be able to detect this change in the electrical properties, a counter electrode is applied to the porous substrate, which allows to apply a potential to the working electrode. The counter electrode can be applied to the substrate together with the working electrode. Alternatively, the counter electrode can be applied to the substrate in a separate step. The application of the counter electrode on the substrate can also be done after the capture molecules have been deposited onto or into the substrate. Due to the deposition of the capture molecules after the application of the working electrode, the capture molecules do not suffer from high and uncontrolled temperature and/or pressure during the application of the working electrode. Also, the exact position of the working electrode is less critical as the deposition of the capture molecules can be adjusted to the exact position of the working electrode. In summary, this leads to a more controlled and more reproducible method for producing an electrochemical lateral flow test device. As the capture molecules are only deposited where they contribute to a signal that can be measured by the working electrode, namely in the direct vicinity of the working electrode, the amount of capture molecules needed can be reduced. Preferably at least a part of the capture molecules is in contact with the working electrode. Optionally the capture molecules are within a distance of 1 mm or less to the working electrode.

In addition, the porous substrate might comprise a sample pad, onto or into which the sample can be deposited in order to test the sample. The sample pad might contain substrate molecules that enhance or enable the chemical reaction at or near the working electrode eventually. In order to perform a test, the sample might for example be placed onto or into the sample pad, where the substrate molecules will be mobilized by the sample. Due to porous flow (or capillary flow) though the porous substrate, the sample together with the mobilized substrate molecules are transported to an (optional) conjugate pad, where the biomarker - if present in the sample - reacts with conjugate molecules, thereby forming intermediates. The molecules are further transported by the capillary flow to the working electrode, where a chemical reaction involving the intermediates, the substrate molecules and the capture molecules takes place. This reaction may be an enzymatic reaction. It leads to a change of the chemical composition in the environment next to the working electrode. The reaction cannot take place if the sample does not comprise the biomarker, hence the electrochemical lateral flow device can discriminate between samples with and without the biomarker. Alternatively, the sample can be mixed with the substrate molecules and/or the conjugate molecules prior to the application onto or into the porous substrate of the electrochemical lateral flow device.

Optionally, at least the working electrode and the counter electrode are applied on the porous substrate by printing, in particular by screen printing. Printing in general and specifically screen printing offers the possibility to apply the electrodes in a reproducible and cost-efficient manner. Screen printing the electrodes on the porous substrate of an electrochemical lateral flow devices with previously applied capture molecules could damage the capture molecules due to high temperatures during the screen printing process. Thus, the capability of the device to detect a biomarker could be destroyed or at least deteriorated. It is therefore foreseen by the present disclosure to deposit the capture molecules only after the working electrode has been applied to the substrate.

In an optional embodiment at least one additional working electrode and/or a reference electrode is applied on the porous substrate. The implementation of a reference electrode allows electrochemical measurements in the commonly used three-electrode-configuration, with current flowing through the working electrode and the counter electrode and the potential of the working electrode can be applied with respect to the reference electrode. Alternatively or additionally, an additional working electrode can be implemented, which can be used to target a second biomarker in the sample or to test the presence of the conjugate molecules as a validation for the result obtained for the first biomarker by detecting false negatives. The lateral flow test device may be configured to detect two different species of biomarkers by applying at least two working electrodes and depositing two species of capture molecules. Optionally, also the conjugate molecules comprise at least two species that target at least two different biomarkers. Optionally at least three working electrodes can be applied, which can be used to target at least three different biomarkers in the sample.

Preferably, the working electrode comprises at least one electrode opening, wherein the capture molecules are deposited onto or into the substrate through the at least one electrode opening. The electrode opening can reach from a top side of the working electrode to a bottom side of the working electrode. The bottom side of the working electrode is attached to the porous substrate, for example to a top side of the porous substrate. The electrode opening can be the access opening. The porous substrate comprises a bottom side opposite of the top side. By applying the capture molecules onto or into the porous substrate through the at least one electrode opening, the capture molecules can be deposited next to or in the vicinity of the working electrode in a particularly reproducible and simple way.

Optionally, the working electrode may be porous, enabling capillary flow of the capture molecules through the porous working electrode, and depositing the capture molecules comprises depositing the capture molecules onto the porous working electrode. By applying the capture molecules onto or into the porous substrate through porous working electrode, the capture molecules can be deposited onto or into the substrate in the vicinity of the working electrode in a reproducible and simple way.

Further optionally, the capture molecules may be deposited onto a bottom side of the substrate, wherein the bottom side of the substrate is facing away from the working electrode. The capture molecules can be deposited on the bottom side of the porous substrate opposite of the working electrode. For example, the capture molecules may be deposited in a deposition area of the bottom side of the substrate, which deposition area fully overlaps with the area defined by the working electrode on the opposite side of the substrate. The porous substrate might have a height or thickness of 0.02 mm to 1 mm.

In an optional embodiment the bottom side of the substrate comprises a microfluidic barrier, wherein the microfluidic barrier extends essentially over the total bottom side of the substrate, wherein the microfluidic barrier comprises at least one barrier opening in a region opposite the working electrode, wherein the capture molecules are deposited onto the porous substrate through the at least one barrier opening. The bottom side of the substrate is facing away from the working electrode, wherein the at least one access opening is the barrier opening provided in the microfluidic barrier. Optionally the microfluidic barrier extends at least over the area of the substrate penetrated by the sample during use of the electrochemical lateral flow test device. The microfluidic barrier can prevent the sample from leaking out of the bottom side of the substrate. If the bottom side of the substrate comprises a microfluidic barrier, there can be a barrier opening extending from the bottom side of the microfluidic barrier to the top side of the microfluidic barrier, whereas the top side of the microfluidic barrier is in contact with the bottom side of the porous substrate. The barrier opening can be the access opening. Similar to the deposition of the capture molecules through an electrode opening, the deposition of the capture molecules can be done through the barrier opening, thus leading to a particularly reproducible and simple way of depositing the capture molecules.

Preferably, at least the working electrode and the counter electrode are printed electrodes. Printing in general, and specifically screen printing, offers the possibility to apply the electrodes on the porous substrate in a reproducible and cost-efficient manner. Especially screen printing could damage previously applied capture molecules due to high temperatures during the screen printing process, thus destroying the capabilities of the device to detect a biomarker. It is therefore foreseen in this disclosure to deposit the capture molecules only after the working electrode has been applied to the substrate.

Preferably, the at least one access opening is an electrode opening provided in the working electrode. The electrode opening can reach from a top side of the working electrode to a bottom side of the working electrode, whereas the bottom side of the working electrode is attached to the porous substrate, specifically to a top side of the porous substrate. The porous substrate comprises a bottom side opposite of the top side. By applying the capture molecules onto or into the porous substrate through the at least one electrode opening, the capture molecules can be deposited next to or in the vicinity of the working electrode in a particularly reproducible and simple way.

By way of example, the disclosure is further explained with respect to some selected embodiments shown in the drawings for purposes of illustration. However, these embodiments shall not be considered limiting for the disclosure.

Fig. 1A-C schematically illustrate a prior art, conventional lateral flow test device with a readout in form of a change in color of a test line.

Figs. 2A-D schematically illustrate the basic principle of operation of an electrochemical lateral flow test device in different stages of a performed test.

Figs. 3A-D schematically illustrate another example of an electrochemical lateral flow test device with a substrate pad in different stages of a performed test.

Figs. 4A-C show an embodiment of the electrochemical lateral flow test device according to the present disclosure with electrode openings in four working electrodes.

Figs. 4D-E show the deposition of capture molecules onto the porous substrate through the electrode openings to receive the electrochemical lateral flow test device of figs. 4A-C.

Fig. 5 shows another electrochemical lateral flow test device according to the present disclosure, similar to Fig. 4A, only with wider electrode openings.

Fig. 6 shows another electrochemical lateral flow test device according to the present disclosure with a plurality of electrode openings per working electrode.

Figs. 7A-C show another embodiment of the electrochemical lateral flow test device according to the present disclosure with barrier openings.

Figs. 7D-E show the deposition of capture molecules onto the porous substrate through the barrier openings to receive the electrochemical lateral flow test device of figs. 7A-C.

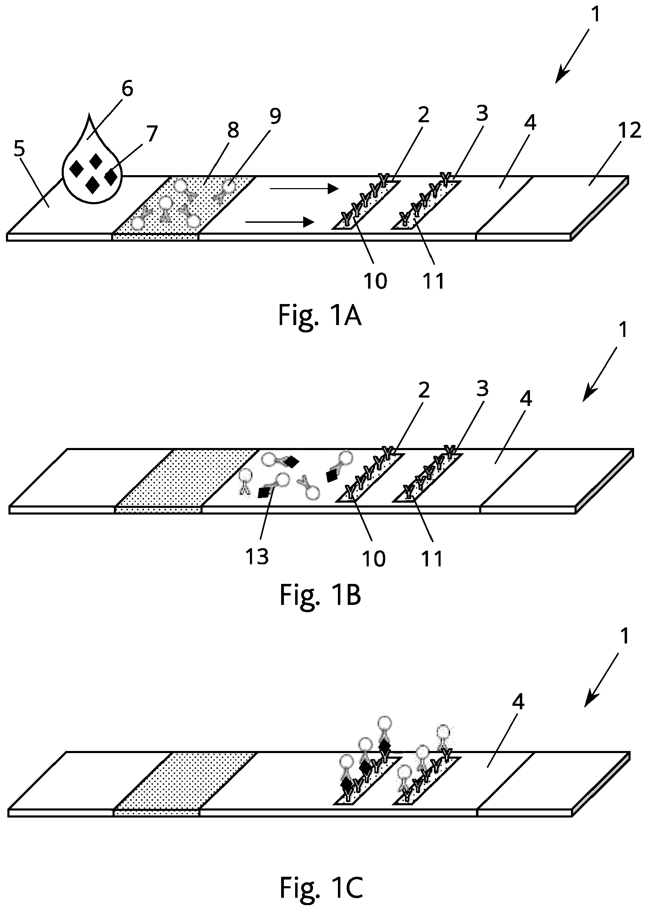

Fig. 1A shows a conventional lateral flow test device 1 with a readout in form of a change in color of a test line 2 and a control line 3 on a porous substrate 4. The substrate comprises a sample pad 5, which is configured to receive a sample 6, which sample 6 can contain a biomarker 7. The substrate 4 further comprises a conjugate pad 8 containing conjugate molecules 9, that might for example contain an antibody to the biomarker 7 and hence bind to the biomarker 7 forming intermediates 13 (see Fig. 1B). Additionally, the substrate 4 comprises a test line 2 with first capture molecules 10 and the control line 3 with second capture molecules 11. An absorbent pad 12 is provided, which is configured to soak up excessive sample 6 after a test has been performed.

Fig. 1B shows the conventional lateral flow device 1 after the sample 6 has been applied to the sample pad 5. By means of capillary flow through the substrate 4, the sample 6 is transported through the conjugate pad 8, thereby interacting with and mobilizing the conjugate molecules 9. The biomarker 7 binds to the conjugate molecules 9 thus forming intermediates 13, which are transported further towards the test line 2 and the control line 3.

Fig. 1C shows the conventional lateral flow test device 1 of Fig. 1A and Fig. 1B after the test has been conducted. The intermediates 13 consisting of the biomarker 7 and conjugate molecules 9 react with first capture molecules 10 at the test line. This results in an accumulation of intermediates 13 at the test line. The conjugate molecules 9 that are part of the accumulated intermediates 13 change the color of the test line 2, for example from white to red. The conjugate molecules 9 can for example comprise gold nano particles. Conjugate molecules 9, which did not react with biomarkers 7, were transported to the control line 3. There, second capture molecules 11 react with conjugate molecules 9. This leads to an accumulation of conjugate molecules 9 at the control line 3. The accumulated conjugate molecules 9 change the color of the control line 3, for example from white to red. Excessive sample 6 is absorbed by the substrate 4 and in particular by the absorbent pad 12. In this example, the relevant biomarker 7 is present in the sample 6, thus the test line 2 as well as the control line 3 change color. The test is therefore valid because the control line 3 has changed color, and positive because the test line 2 also changed color. The readout can be done by a user for example by visual inspection.

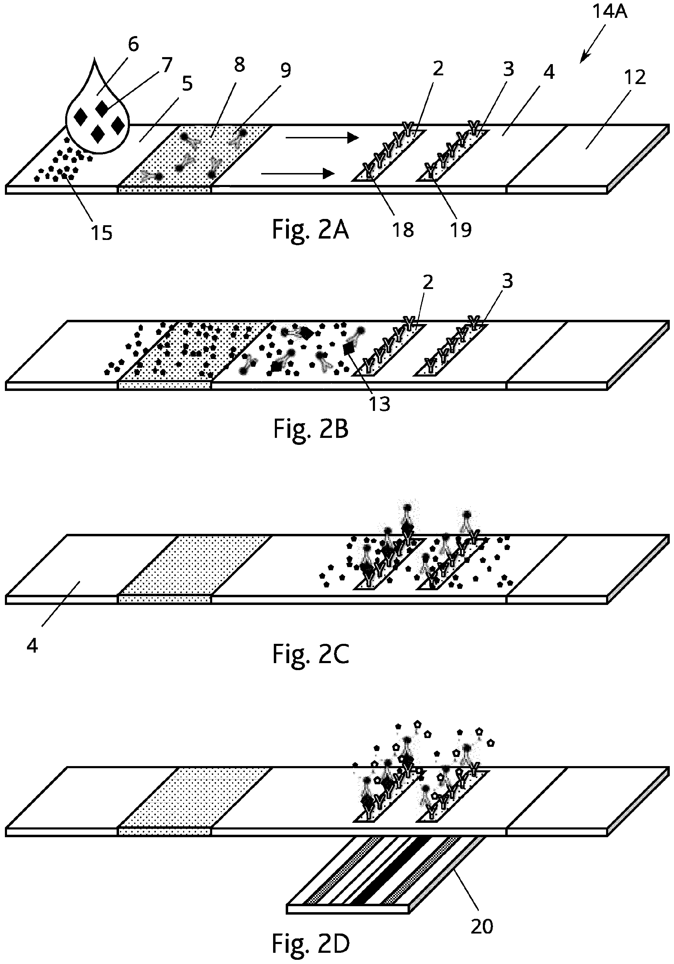

Fig. 2A shows a porous substrate 4 for an electrochemical lateral flow test device 14A. In contrast to the conventional lateral flow test device 1 of figs. 1A-D, there is no change in color to readout the result of a test but a chemical reaction leading to a change in electric properties in the vicinity of a test line 2 and a control line 3. Compared to the conventional/visual lateral flow test device 1 shown in Figs. 1A-C, substrate 4 comprises immobilized substrate molecules 15 in the sample pad 5. At the test line 2 there are capture molecules 18, which were deposited onto or into the substrate 4 at the control line 3. There are further capture molecules 19, which were deposited onto or into the substrate 4. The species of conjugate molecules 9 as well as the capture molecules 18 and the further capture molecules 19 are adjusted to the targeted biomarker 7. The substrate molecules 15 are adjusted to an enzyme comprised by the conjugate molecules 9.

Fig. 2B shows the substrate 4 of fig. 2A. The sample 6 has been applied to the sample pad 5, thereby mobilizing the substrate molecules 15. By means of capillary flow through the substrate 4, the sample 6 together with the substrate molecules 15 is transported through the conjugate pad 8, thereby interacting with and mobilizing the conjugate molecules 9. The biomarker 7 binds to the conjugate molecules 9 thus forming intermediates 13, which are transported further towards test line 2 and the control line 3.

Fig. 2C shows substrate 4 of Fig. 2A and Fig. 2B after the chemical reactions involving the biomarker 7 have taken place. The intermediates 13 consisting of the biomarker 7 and conjugate molecules 9 react with capture molecules 18 and substrate molecules 15 at the test line 2, thus changing the electrical properties of the test line 2 within the substrate 4. Conjugate molecules 9, which did not bind with biomarkers 7, were transported to the control line 3. There, further capture molecules 19 react with the conjugate molecules 9 and substrate molecules 15, thus changing the electrical properties of control line 3 within the substrate 4. More in detail, the change in electrical properties may be due to ongoing enzymatic reactions mediated by the captured molecules until the substrate molecules are consumed. Excessive sample 6 is absorbed by the substrate 4 and in particular by the absorbent pad 12. In this example, the relevant biomarker 7 is present in the sample 6, thus there is a chemical reaction at the test line 2.

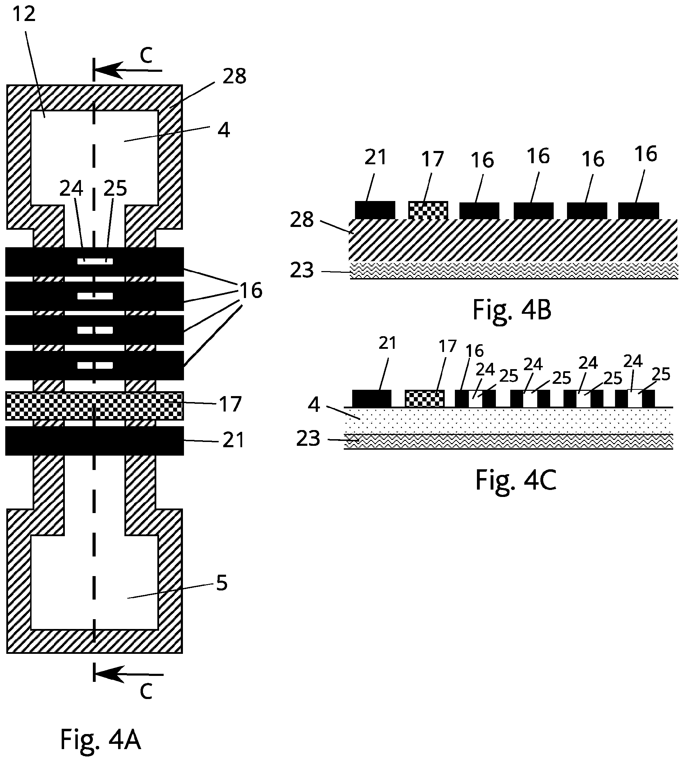

Fig. 2D shows the substrate 4 of figs. 2A-C after the test has been conducted. In order to read-out the result, preferably during the test is conducted, in this example a separate structure 20 with electrodes (not shown) is applied to the substrate 4 of fig. 2C. These electrodes can be used to quantify the change in the electrical properties of their environment, which is in this case the test line 2 and the control line 3. The structure 20 can for example comprise a working electrode for measuring the test line 2, an additional working electrode for measuring the control line 3 as well as a counter electrode, which allows to apply a potential to the working electrodes. By applying electrodes in a separate structure 20 after the capture molecules 18 have been deposited onto or into the substrate 4, the capture molecules can be damaged. In that case, the electrochemical lateral flow test device 14A of figs. 2A-D might yield non-reproducible results. However, the same working principle shown in Fig. 2A-D applies to the devices shown from Fig. 4A onward, which avoid this disadvantage.

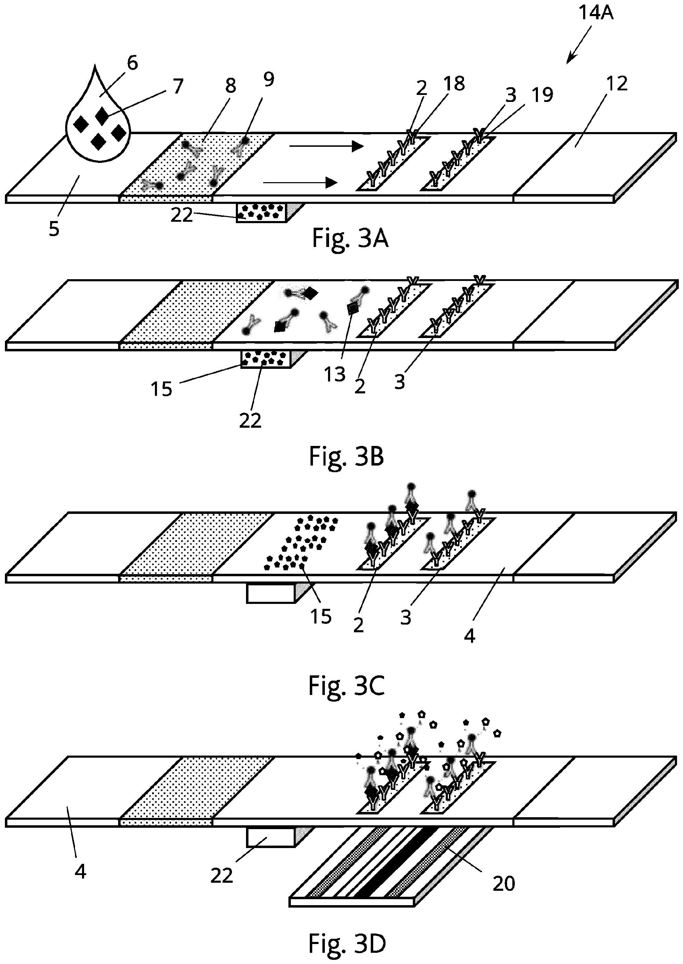

Fig. 3A shows another example of an electrochemical lateral flow test device 14A. Compared to the example of figs. 2A-D, the substrate 4 comprises a substrate pad 22, which contains the substrate molecules 15 instead of the sample pad 5. The substrate pad 22 is a separate porous material fixed to the substrate 4. Alternatively, the substrate pad 22 can be part of the substrate 4. As shown in Fig. 3B-D, the substrate molecules 15 are only mobilized by the sample 6 after the sample 6 has passed the conjugate pad 8, leading to a delayed release of the substrate molecules 15 and therefore for example a delayed enzymatic reaction.

Fig 4A shows a top view of an embodiment of an electrochemical lateral flow test device 14 according to the present disclosure. The principle of operation is essentially the same as shown in figs. 2A-D. The main difference is that the working electrodes 16 have been applied to the substrate 4 before capture molecules 18 have been deposited onto the substrate 4. The substrate 4 comprises a sample pad 5 as well as an absorbent pad 12. The substrate 4 is surrounded by a lateral microfluidic barrier 28, which consists of a hydrophobic material. The lateral microfluidic barrier 28 guides the sample 6 and prevents the sample 6 from leaking out. Additionally, there are four working electrodes 16, a reference electrode 17 and a counter electrode 21 applied on the substrate 4. The working electrodes 16 each comprise an electrode opening 24, which serves as an access opening 25. Different types of capture molecules 18 (see figs. 4C-D) have been applied into or onto the substrate 4 through the electrode openings 24 after the working electrodes 16 have been applied to the substrate 4. The working electrodes 16, the reference electrode 17 as well as the counter electrode 21 can be connected to a measurement device (not shown) to read out the result. The measurement device can for example be a multimeter or a micro-controller. The measurement can for example be done by chrono-amperometry.

Fig. 4B shows a side view of the electrochemical lateral flow test device 14 of Fig. 4A. There is a microfluidic barrier 23 on the bottom side of the substrate 4, which extends over the whole bottom side of the substrate 4. Alternatively, the microfluidic barrier 23 can extend at least over the area of the substrate 4 penetrated by the sample 6 during use of the electrochemical lateral flow test device 1. This area can for example be limited by the lateral microfluidic barrier 28.

Fig. 4C shows a cross section of the electrochemical lateral flow test device 14 of Figs. 4A and 4B along the cutting line C-C of Fig. 4A. The electrode openings 24 extend from the top side of the working electrodes 16 to the bottom side of the working electrodes 16. The capture molecules 18 (see Fig.2D-E) have been deposited onto the substrate 4 through the working electrode openings 24.

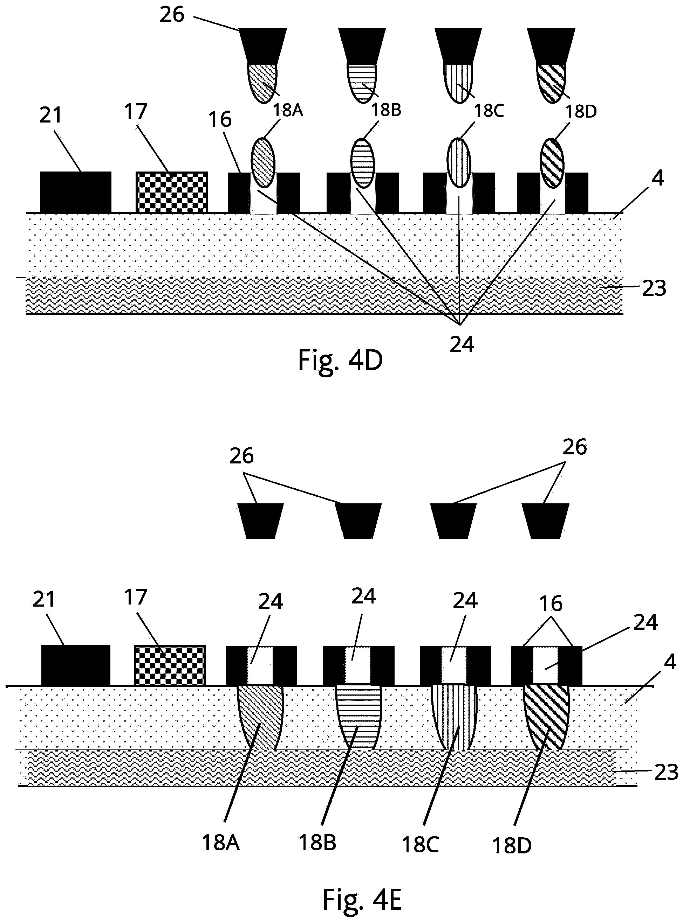

Fig. 4D shows the cross section of Fig. 4C during the process of depositing the capture molecules 18 through the electrode openings 24 to receive the electrochemical lateral flow test device 14. In this example, printer nozzles 26 are used to deposit the capture molecules 18 onto the substrate 4 in a reproducible and controlled way. The capture molecules 18A, 18B, 18C and 18D may all be the same species or a different species to target up to four different biomarkers 7. In case the capture molecules 18A, 18B, 18C and 18C are different species, also corresponding species of conjugate molecules 9 may be used to target different species of biomarkers 7.

Fig. 4E shows the same cross section as Fig. 4D after the capture molecules 18A, 18B, 18C, 18D have been deposited onto the substrate 4. The capture molecules 18A, 18B, 18C, 18D have flowed and/or diffused into the substrate 4 below and in the vicinity of the respective working electrode 16. The microfluidic barrier 23 on the bottom side of the substrate 4 prevented the capture molecules 18A, 18B, 18C and 18D from leaking out of the substrate 4.

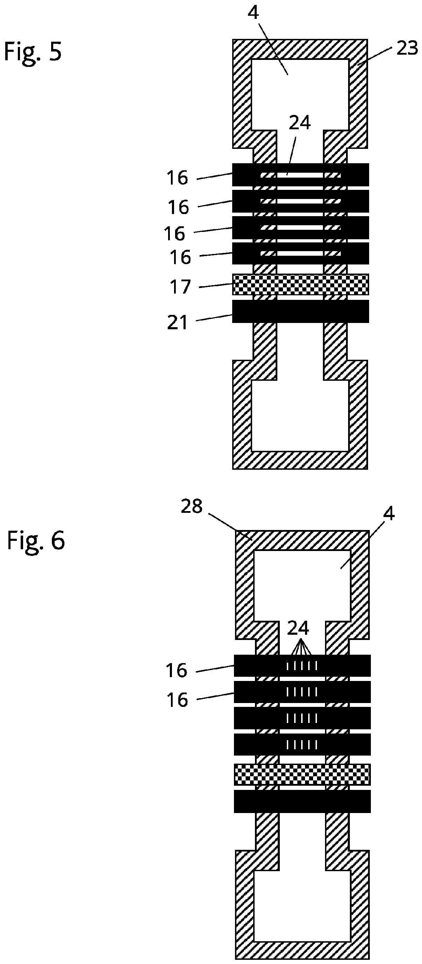

Fig. 5 shows a top view of another embodiment of an electrochemical lateral flow test device 14, similar to Fig. 4A. In contrast to the embodiment of Fig. 4A, the electrode openings 24 are larger relative to the working electrodes 16 compared to Fig. 4A. The working electrodes 16 and the counter electrode 21 as well as the reference electrode 17 have been applied via printing.

Fig. 6 shows a top view of a third embodiment of an electrochemical lateral flow test device 14, similar to Figs. 4A and 5. In contrast to the embodiment of Figs. 4A and 5, the working electrodes 16 each comprise a plurality of, specifically five, spaced apart electrode openings 24.

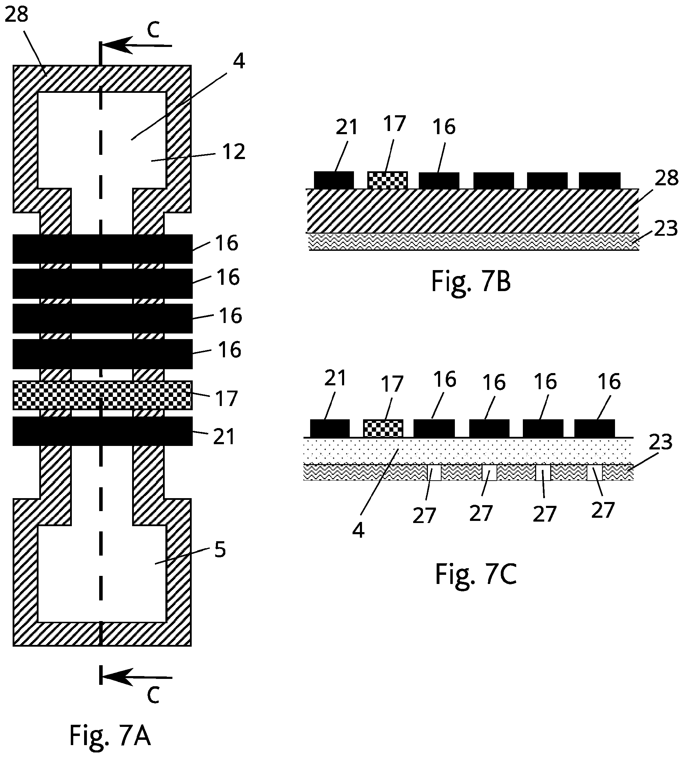

Fig. 7A shows a top view of a fourth embodiment of an electrochemical lateral flow test device 14. The substrate comprises a sample pad 5 as well as an absorbent pad 12. The substrate 4 is surrounded by a lateral microfluidic barrier 28, which consists of a hydrophobic material. The lateral microfluidic barrier 28 guides the sample 6 and prevents the sample 6 from leaking out. Additionally, there are four working electrodes 16, a reference electrode 17 and a counter electrode 21 applied on the substrate 4. Capture molecules 18 have been applied into or onto the substrate 4 through barrier openings 27 (see Fig. 7C) from the bottom side of the substrate 4.

Fig. 7B shows a side view of the electrochemical lateral flow test device 14 of Fig. 7A. It can be seen, that there is a microfluidic barrier 23 on the bottom side of the substrate 4, which extends basically over the whole bottom side of the substrate 4. The bottom side of the substrate 4 is facing away from the working electrodes 16.

Fig. 7C shows a cross section of the electrochemical lateral flow test device 14 of Figs. 7A and 7B along the cutting line C-C of Fig. 7A. The microfluidic barrier 24 on the bottom side of the substrate 4 comprises barrier openings 27 in a region opposite the working electrodes 16 extending all the way through the microfluidic barrier 23 of the bottom side of the substrate 4.

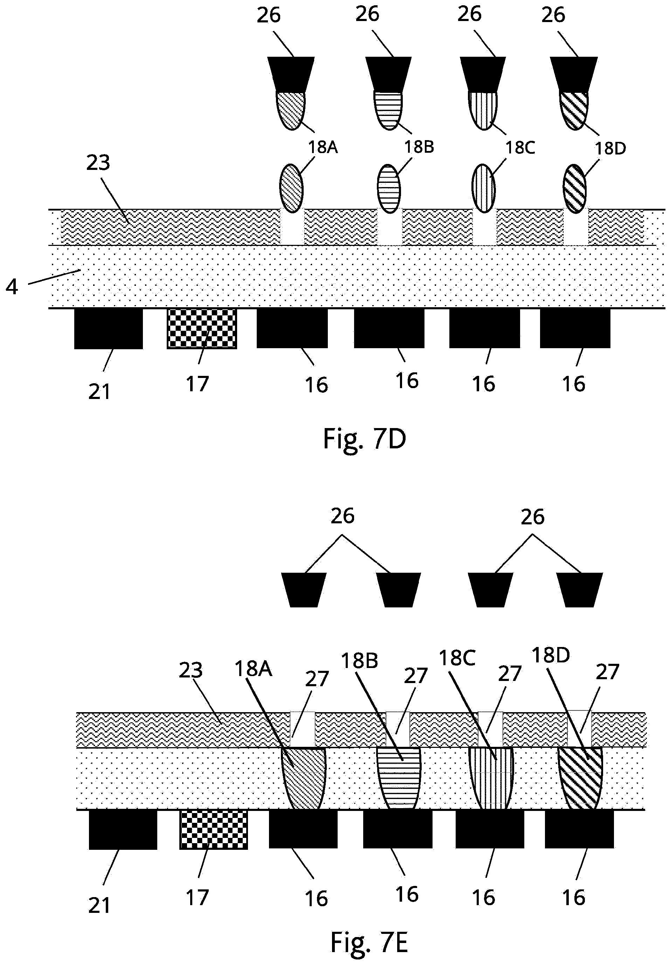

Fig. 7D shows an upside-down view of the cross section of Fig. 7C during the process of depositing the capture molecules 18 through the barrier openings 24 to receive the electrochemical lateral flow test device 14. Printer nozzles 26 are used to deposit the capture molecules 18 onto the substrate 4 in a reproducible and controlled way. The capture molecules 18A, 18B, 18C and 18D may be the same kind or a different kind to target up to four different biomarkers 7.

Fig. 7E shows the same cross section as Fig. 7D after the capture molecules 18A, 18B, 18C, 18D have been deposited onto the substrate 4 through the barrier openings 27. The capture molecules 18A, 18B, 18C, 18D have flown and diffused into the substrate 4 below and in the vicinity of the respective working electrode 16. The microfluidic barrier 23 on the bottom side of the substrate 4 prevented the capture molecules 18A, 18B, 18C, 18D from leaking out of the substrate 4.

## Claims

1. A method for producing an electrochemical lateral flow test device (14) for the detection of a biomarker (7), the method comprising the steps:
- providing a porous substrate (4) enabling a capillary flow of a sample (6) comprising the biomarker (7) through the porous substrate (4);
- applying at least a working electrode (16) and a counter electrode (21) on the porous substrate (4); and
- depositing capture molecules (18, 18A, 18B, 18C, 18C) onto the substrate (4) next to the applied working electrode (16).

2. The method according to claim 1, **characterized by** applying at least the working electrode (16) and the counter electrode (21) on the porous substrate (4) by printing, in particular by screen printing.

3. The method according to claim 1 or 2, **characterized by** applying at least one additional working electrode (16) and/or a reference electrode (17) on the porous substrate (4).

4. The method according to any one of claims 1 to 3, **characterized in that** the working electrode (16) comprises at least one electrode opening (24), wherein the capture molecules (18, 18A, 18B, 18C, 18D) are deposited onto or into the substrate (4) through the at least one electrode opening (24).

5. The method according to any one of claims 1 to 3, **characterized in that** the working electrode (16) is porous, enabling capillary flow of the capture molecules (18, 18A, 18B, 18C, 18D) through the porous working electrode (16), and **in that** depositing the capture molecules (18, 18A, 18B, 18C, 18D) comprises depositing the capture molecules (18, 18A, 18B, 18C, 18D) onto the porous working electrode (16).

6. The method according to any one of claims 1 to 3, **characterized by** depositing the capture molecules (18, 18A, 18B, 18C, 18D) onto a bottom side of the substrate (4), wherein the bottom side of the substrate (4) is facing away from the working electrode (16).

7. The method according to claim 6, **characterized in that** the bottom side of the substrate (4) comprises a microfluidic barrier (23), wherein the microfluidic barrier (23) extends essentially over the total bottom side of the substrate (4), wherein the microfluidic barrier (23) comprises at least one barrier opening (27) in a region opposite the working electrode (16), wherein the capture molecules (18, 18A, 18B, 18C, 18D) are deposited onto or into the porous substrate (4) through the at least one barrier opening (27).

8. An electrochemical lateral flow test device (14) for the detection of a biomarker (7), the electrochemical lateral flow test device (14) comprising:
- a porous substrate (4) enabling a capillary flow of a sample (6) comprising the biomarker (7) through the porous substrate (4),
- at least a working electrode (16) and a counter electrode (21) for electrochemical detection arranged on the porous substrate (4),
wherein the porous substrate (4) comprises capture molecules (18, 18A, 18B, 18C, 18D),
**characterized in that** the electrochemical lateral flow test device (14) further comprises at least one access opening (25) for depositing the capture molecules (18, 18A, 18B, 18C, 18D) onto the porous substrate (4) next to the previously applied working electrode (16) through the at least one access opening (24) during production of the electrochemical lateral flow test device (14).

9. The electrochemical lateral flow test device (14) according to claim 8, **characterized in that** at least the working electrode (16) and the counter electrode (21) are printed electrodes.

10. The electrochemical lateral flow test device (14) according to claim 8 or 9, **characterized in that** electrochemical lateral flow test device (14) further comprises a reference electrode (17) arranged on the substrate (4).

11. The electrochemical lateral flow test device (14) according to any one of claims 8 to 10, **characterized in that** the at least one access opening (25) is an electrode opening (24) provided in the working electrode (16).

12. The electrochemical lateral flow test device (14) according to any one of claims 8 to 10, **characterized in that** the electrochemical lateral flow test device (14) further comprises a microfluidic barrier (23) arranged on the bottom side of the substrate (4), wherein the bottom side of the substrate (4) is facing away from the working electrode (16), wherein the at least one access opening (25) is a barrier opening (27) provided in the microfluidic barrier (23).
